# EUROPEAN PATENT SPECIFICATION

(11) **EP 2 108 045 B1**
(45) Date of publication and mention of the grant of the patent: **11.12.2013**
(21) Application number: 08707385.4
(22) Date of filing: 29.01.2008
(51) Int. Cl.: C12N 9/64

(54) **IMPROVED FIX-MUTANT PROTEINS FOR HEMOPHILIA B TREATMENT**
VERBESSERTE FIX-MUTANTEN-PROTEINE ZUR BEHANDLUNG VON HÄMOPHILIE B
PROTÉINES MUTANTES DE FIX AMÉLIORÉES DESTINÉES À TRAITER L'HÉMOPHILIE B

(30) Priority: 01.02.2007 US 898876 P
(43) Date of publication of application: 14.10.2009
(73) Proprietor: Baxter International Inc., Deerfield, IL 60015 (US); Baxter Healthcare SA, 8152 Glattpark (Opfikon) (CH)
(72) Inventor: DOCKAL, Michael, A-1150 Vienna (AT); HARTMANN, Rudolf, 2102 Bisamberg (AT); SCHEIFLINGER, Friedrich, A-1090 Vienna (AT)
(74) Representative: Müller-Boré & Partner Patentanwälte
(86) International application number: PCT/EP2008/000684
(87) International publication number: WO 2008/092643

(56) References cited:
- WO-A-99/03496
- CHANG YU-JIA ET AL: "Identification of functionally important residues of the epidermal growth factor-2 domain of factor IX by alanine-scanning mutagenesis. Residues Asn(89)-Gly(93) are critical for binding factor VIIIa" JOURNAL OF BIOLOGICAL CHEMISTRY, AL, vol. 277, no. 28, 12 July 2002 (2002-07-12), pages 25393-25399, XP002464800 ISSN: 0021-9258
- JING-BIN YAN ET AL: "Transgenic Mice Can Express Mutant Human Coagulation Factor IX with Higher Level of Clotting Activity" BIOCHEMICAL GENETICS, KLUWER ACADEMIC PUBLISHERS-PLENUM PUBLISHERS, NE, vol. 44, no. 7-8, 21 September 2006 (2006-09-21), pages 347-358, XP019447249 ISSN: 1573-4927
- DATABASE BIOSIS [Online] BIOSCIENCES INFORMATION SERVICE, PHILADELPHIA, PA, US; 16 November 2002 (2002-11-16), LIN SHU-RUNG ET AL: "Identification of Functionally Important Residues in the Protease Domain of Factor IX That Are Critical for Binding Factor XIa, TFPI, and Antibodies." XP009101499 Database accession no. PREV200300335606 & BLOOD, vol. 100, no. 11, 16 November 2002 (2002-11-16), page Abstract No. 1006, 44TH ANNUAL MEETING OF THE AMERICAN SOCIETY OF HEMATOLOGY; PHILADELPHIA, PA, USA; DECEMBER 06-10, 2002 ISSN: 0006-4971
- CHANG JINLI ET AL: "Changing residue 338 in human factor IX from arginine to alanine causes an increase in catalytic activity" JOURNAL OF BIOLOGICAL CHEMISTRY, AL, vol. 273, no. 20, 15 May 1998 (1998-05-15), pages 12089-12094, XP002199691 ISSN: 0021-9258
- SICHLER KATRIN ET AL: "Physiological fIXa activation involves a cooperative conformational rearrangement of the 99-loop." JOURNAL OF BIOLOGICAL CHEMISTRY, vol. 278, no. 6, 7 February 2003 (2003-02-07), pages 4121-4126, XP002484064 ISSN: 0021-9258
- HARTMANN RUDOLF ET AL: "Variants of recombinant factor IX with enhanced functional properties" BLOOD, vol. 110, no. 11, Part 1, November 2007 (2007-11), page 791A, XP009101449 & 49TH ANNUAL MEETING OF THE AMERICAN-SOCIETY-OF-HEMATOLOGY; ATLANTA, GA, USA; DECEMBER 08 -11, 2007 ISSN: 0006-4971

## Description

The present invention relates to a recombinant blood coagulation factor IX (rFIX) mutant having improved FIX clotting potential, cell cultures expressing the rFIX mutant, and a pharmaceutical composition for treating a bleeding disorder comprising said rFIX mutant.

The blood coagulation cascade involves a series of serine protease enzymes (zymogens) and protein cofactors. When required, an inactive zymogen precursor is converted into the active form, which consequently converts the next enzyme in the cascade.

The cascade is divided into three distinct segments: the intrinsic, extrinsic, and common pathways (Schenone et al., Curr Opin Hematol. 2004;11:272-7). The activation of factor X (FX) is the common point of the intrinsic and extrinsic pathways. The activation occurs either by the extrinsic complex formed by activated factor VII (FVlla) and tissue factor, or by the intrinsic tenase complex composed of activated Factor IXa (FIXa) and activated Factor VIIIa (FVllla) (Mann, Thromb. Haemostasis 1999;82:165-74).

Activated FX along with phospholipids, calcium, and factor Va (FVa) then converts prothrombin to thrombin (prothrombinase complex), which in turn cleaves fibrinogen to fibrin monomers. The monomers polymerize to form fibrin strands. Factor XIIIa (FXIIIa) covalently bonds these strands to one another to form a rigid mesh.

Deficiencies of the components of the intrinsic tenase complex, FVIIIa and FIXa, lead to severe bleeding disorders, hemophilia A and B, respectively. Hemophilia A is considered the classic form of hemophilia, whereas hemophilia B is also known as Christmas disease. Hemophilia A and B are the consequence of congenital deficiencies of FVIII and FIX, respectively. The worldwide incidence of hemophilia A is approximately 1 case per 5,000 male individuals and of hemophilia B 1 case per 30,000.

FIXa is a two-chain vitamin K-dependent serine protease capable of hydrolysing the Arg194-Ile195 peptide bond in the FX molecule which leads to its activation (Venkateswarlu et al., Biophys. J. 2002;82:1190-206). Although this reaction can proceed slowly in solution, it is significantly accelerated in the presence of negatively charged phospholipid surfaces. *In vivo,* these surfaces are mainly provided by activated platelets and plasma lipoproteins. The rate of the reaction is increased further by the presence of FVllla.

Originally patients with hemophilia had a shortened lifespan and diminished quality of life that was greatly affected by hemophilic arthropathy. Nowadays the treatment of choice for the management of hemophilia B is replacement therapy with various plasma derived FIX concentrates or one commercially available rFIX which is BeneFIX® manufactured by Genetics Institute. Although progress in the production of FIX to ensure purity, efficacy and viral safety has been made over the past decades, some limitations remain.

Therapeutic polypeptide drugs such as blood coagulation proteins including FIXa are rapidly degraded by proteolytic enzymes and neutralized by antibodies. This reduces their half-life and circulation time, thereby limiting their therapeutic effectiveness. Relatively high doses and frequent administration are necessary to reach and sustain the desired therapeutic or prophylactic effect of FIXa. As a consequence adequate dose regulation is difficult to obtain and the need of frequent intravenous administrations imposes restrictions on the patient's way of living. Thus a FIXa molecule with improved clotting activity could decrease the dosage or the number of necessary administrations.

From FIXa several amino acid residues are already known to be important for its activity and several approaches have already been tried to improve FIX.

For the interaction of FIX with FX several important regions and amino acids have been described. The surface loop 99 of FIXa is important for regulation of FIXa activity (Hopfner et al., Structure Fold Des. 1999;7:989-96). In the non-complexed FIXa this loop is stabilized in an inactive conformation and limits access of substrate to the catalytic machinery.

The mutations Y94F and K98T are located on the 99-loop, known to contribute to FX substrate binding by forming the recognition site of the S2 and S4 pockets of FX. Y177F mimics the effect of activation by FVllla. Tyr 177 locks the 99-loop in an inactive conformation, which is released by binding of FVIIIa to FIXa (Sichler et al., J Biol Chem. 2003; 278:4121-26).

Val 213 and Gly 219 are conserved amino acids in most other trypsin-like proteases, and a double mutant of truncated FIX (I213V-E219G) expressed in *E. coli* showed increased amidolytic activity of FIXa (Hopfner et al., EMBO J. 1997;16:6626-35).

However, in none of these publications full length FIX mutants expressed in mammalian cells showed an improved functional activity, especially in a meaningful activated partial thromboplastin time (aPTT) assay in FIX-depleted plasma.

Another approach was the modification of FIX amino acid residues important for interaction with inhibitors of FIX. Because endogenous heparan sulfate/heparin inhibits the activity of FIX WO0240544 disclosed mutant FIX proteins comprising mutations in the heparin binding domain of FIX (preferably at amino acid residues 170 and 233) which decreased its affinity for heparin. Similarly US6531298 disclosed mutant FIX molecules designed to locate the residues of FIXa that bind FVIIIa. Due to an amino acid substitution at Arg 338 FIX had increased clotting activity and also an reduced affinity for heparin (Chang et al. J Biol Chem. 1998;273:12089-94). In these disclosures combinations of mutations of amino acids important for functional activity of FIX have never been tried.

In this context, Chang *et al.* (Chang, Y.-J. et al., Journal of Biological Chemistry, 227(28), 2002, pp. 25393-25399) describes the identification of functionally important residues of the epidermal growth factor-2 domain of FIX by alanine-scanning mutagenesis. Further, WO 99/03496 A1 describes a non-naturally occurring FIX protein having an amino acid substitution at position 338. Further, Yan *et al.* (Yan, J.-B. et al., Biochemical Genetics, 44(7-8), 2006, pp. 347-358) describes the expression of a FIX protein having a mutation at position 338 in transgenic mice. Furthermore, Lin *et al.* (Lin, S.-R. *et al.,* Biosciences Information Service Database, Database accession no. PREV200300335606) describes the identification of functionally important residues of the catalytic domain of FIX by alanine-scanning mutagenesis. Finally, Hartmann *et al.* (Hartmann, R. et al., Blood, 110(11), 2007, p. 791 A) describes the functional properties of recombinant FIX mutants.

Despite all these efforts there is currently no improved FIX molecule commercially available or in clinical development. Thus, there remains a great need in the art for compositions and methods that provide an improved FIX molecule that can be used for treatment of patients with hemophilia B.

It was the inventive task of the present invention to develop by introduction of combinations of amino acid exchanges novel FIX proteins which have improved FIX clotting activity useful for the treatment of bleeding disorders.

The present invention relates to a recombinant blood coagulation factor IX (rFIX) mutant having improved FIX clotting activity wherein the amino acid sequence of mutated rFIX is SEQ ID NO: 4 (FIX-Y94F/K98T). Three full length FIX proteins with novel combinations of mutations of amino acids important for functional activity of FIX, i.e. FIXY94F/K98T (SEQ ID NO 4), FIX-Y94F/K98T/Y177F (SEQ ID NO 6), FIX-Y94F/K98T/Y177F/I213V/E219G (SEQ ID NO 8) and FIX wild type (SEQ ID NO 2) were cloned, expressed in HEK 293 and purified by a three step purification protocol using anion exchange chromatography, pseudo-affinity chromatography and affinity chromatography. Pre-activated FIX was removed with biotinylated chloromethylketones and streptavidine-sepharose. Among other assays the proteins were tested by an activated partial thromboplastin time (aPTT) assay in FIX-depleted plasma. The functional activity of the FIX mutants was calculated as percentage of specific FIX activity. PdFIX and FIX-WT had specific FIX activities of 97 and 108 %, respectively. FIX-Y94F/K98T/Y177F/I213V/E219G had no higher activity. In contrast to this FIX-Y94F/K98T and FIX-Y94F/K98T/Y177F had activities of 249 and 232 %, respectively. However, when activating the FIX proteins prior to the assay activated FIXa-Y94F/K98T/Y177F/I213V/E219G exhibited a 14.4 fold higher activity then activated pdFIX (pdFIXa), whereas the other mutated proteins showed activities similar to pdFIXa. Therefore the mutated FIX proteins with improved clotting activity can be used for the treatment of bleeding disorders associated with functional defects or deficiencies of FIX.

The present invention relates to a mutated recombinant blood coagulation FIX protein having an improved FIX clotting activity as compared to wild type FIX (FIX-WT) or plasma derived FIX (pdFIX), wherein the amino acid sequence of mutated rFIX is SEQ ID NO: 4 (FIX-Y94F/K98T).

The term "amino acid" within the scope of the present invention is meant to include all naturally occurring L α-amino acids. The one and three letter abbreviations for naturally occurring amino acids are used herein (Lehninger, Biochemistry, 2d ed., Worth Publishers, New York, 1995: 71-92).

The rFIX mutant according to the present invention may be derived from any vertebrate, e.g. a mammal.

According to the present invention, the term "FIX" does not underlie a specific restriction and may include any FIX, with heterologous or naturally occurring sequences, obtained via recombinant DNA technology, or a biologically active derivative thereof. Accordingly, the term "rFIX mutant" includes any recombinant mutant derived from a FIX protein sequence of any of the foregoing FIX. Accordingly, a FIX polynucleotide or polypeptide sequence of the present invention is typically derived from a mammalian FIX sequence including, but not limited to, primate, e.g., human; rodent, e.g., rat, mouse, hamster; cow, pig, horse, sheep, or any other mammalian sequence. In one specific example of the present invention, the rFIX mutant is a recombinant mutant of human FIX. Polynucleotide and polypeptide sequences of the FIX can be found for example in the UniProtKB/Swiss-Prot Accession No. P00740. The mutated rFIX of the invention may be a mutated full length or truncated FIX. In a preferred embodiment of the present invention the mutated rFIX has a full length sequence. In the present invention the chymotrypsinogen numbering within the serine protease domain was used according to Hopfner et al. (EMBO J. 1997;16:6626-35).

A wide variety of vectors can be used for the preparation of a rFIX mutant and can be selected from eukaryotic and prokaryotic expression vectors. Examples of vectors for prokaryotic expression include plasmids such as pRSET, pET, pBAD, etc., wherein the promoters used in prokaryotic expression vectors include lac, trc, trp, recA, araBAD, etc. Examples of vectors for eukaryotic expression include: (i) for expression in yeast, vectors such as pAO, pPIC, pYES, pMET, using promoters such as AOX1, GAP, GAL1, AUG1, etc; (ii) for expression in insect cells, vectors such as pMT, pAc5, pIB, pMIB, pBAC, etc., using promoters such as PH, p10, MT, Ac5, OpIE2, gp64, polh, etc., and (iii) for expression in mammalian cells, vectors such as pSVL, pCMV, pRc/RSV, pcDNA3, pBPV, etc., and vectors derived from viral systems such as vaccinia virus, adeno-associated viruses, herpes viruses, retroviruses, etc., using promoters such as CMV, SV40, EF-1, UbC, RSV, ADV, BPV, and β-actin.

A mutated rFIX according to the present invention may be produced by any method known in the art, for example any method applicable to non-mutated rFIX. An example was first published by Kaufman et al. (J Biol Chem. 1986;261:9622-8). The production of a rFIX mutant may include any method for the generation of recombinant DNA by genetic engineering, e.g. via reverse transcription of RNA and/or amplification of DNA.

A nucleic acid sequence encoding a mutant rFIX protein according to the invention may be generated by any method known in the art. Examples are polymerase chain reaction (PCR) and cloning methods. In a preferred embodiment of the present invention the DNA encoding a mutant protein ot the invention is generated by *in vitro* mutagenesis using specific primers to generate the respective mutations.

Additionally, the recombinant DNA coding for a mutant rFIX according to the present invention, e.g. a plasmid, may also contain a DNA sequence encoding a selectable marker for selecting the cells which have been successfully transfected with the plasmid. In an example of the present invention, the plasmid may also confer resistance to a selectable marker, e.g. to the antibiotic drug hygromycin, by delivering a resistance gene, e.g. the hygromycin resistance gene conferring resistance to the marker.

The production of a rFIX mutant may include any method known in the art for the introduction of recombinant DNA into eukaryotic cells by transfection, e.g. via electroporation or microinjection. For example, the recombinant expression of a human rFIX mutant can be achieved by introducing an expression plasmid containing the human rFIX mutant encoding DNA sequence under the control of one or more regulating sequences such as a strong promoter, into a suitable host cell line by an appropriate transfection method resulting in cells having the introduced sequences stably integrated into the genome. The lipofection method is an example of a transfection method which may be used according to the present invention.

The production of a rFIX mutant may also include any method known in the art for the cultivation of said transformed cells, e.g. in a continuous or batchwise manner, and the expression of the rFIX mutant, e.g. constitutive or upon induction. In one specific example of the present invention the nucleic acid coding for rFIX mutant contained in the host organism of the present invention is expressed via an expression mode selected from the group consisting of induced, transient, and permanent expression. Any expression system known in the art or commercially available can be employed for the expression of a recombinant nucleic acid encoding rFIX mutant , including the use of regulatory systems such as suitable, e.g. controllable, promoters, enhancers etc.

The production of a rFIX mutant may also include any method known in the art for the isolation of the protein, e.g. from the culture medium or by harvesting the transformed cells. For example, the rFIX mutant -producing cells can be identified by isolating single-cell derived populations, i.e. cell clones, via dilution after transfection and optionally via addition of a selective drug to the medium. After isolation the identified cell clones may be cultivated until confluency in order to enable the measurement of the rFIX mutant content of the cell culture supernatant by enzyme-linked immunosorbent assay (ELISA) technique. Additionally, rFIX mutant secreted by the cells may be identified for example by growing the cells in the absence of any growth promoting fetal serum or components thereof. Vitamin K is added at appropriate concentrations to improve the functional properties of the rFIX mutant protein. After identification, high rFIX mutant producing cell clones may for example be further propagated and/or stored via cryopreservation. The rFIX mutant may be also co-expressed with vitamin K reductase complex subunit 1 (VKORC1, Hallgren et al., Biochemistry 2006;45:5587-98) and/or furin (Wasley et al. J Biol Chem. 1993;268: 8458-65).

The host cell type according to the present invention may be any eukaryotic cell. In a preferred embodiment the cell is a mammalian cell with the ability to perform posttranslational modifications of rFIX mutant. For example said mammalian cell is derived from a mammalian cell line, like for example a cell line selected from the group consisting of SkHep-, CHO-, HEK293-, and BHK-cells. In a specific example of the present invention, the rFIX mutant is expressed in HEK293-derived cells.

There is no particular limitation to the media, reagents and conditions used for culturing the cells in the cell culture of the present invention including culturing the cells in a continuous or batch-wise manner. The cells may be cultured also under serum-free or serum- and protein-free conditions. In a specific example of the present invention the cells are cultured in a mixture of Dulbecco's modified Eagle's Medium and F-12 medium.

Additionally, the production of a rFIX mutant may include any method known in the art for the purification of rFIX mutant , e.g. via anion exchange chromatography or affinity chromatography. In one preferred embodiment rFIX mutant can be purified from cell culture supernatants by anion exchange chromatography, tandem-pseudoaffinity and affinity chromatography. The purified rFIX mutant may be analyzed by methods known in the art for analyzing recombinant proteins, e.g. the ELISA technique and by electrophoresis techniques including immuno-blotting.

The term "improved FIX clotting activity" as used herein means the functional activity of a mutant rFIX protein of the present invention and any other mutant rFIX protein which may be assessed for example by measuring activated partial thromboplastin time (aPTT).

The aPTT assays represent meaningful assays for testing the functional activity of a rFIX mutant protein because they measure the clotting time in plasma. In principle the clotting activity of any compound is determined by its addition to plasma samples and measurement of time to clotting. This can be carried out for example in plasma depleted with a protein or in plasma from inhibitor patients.

A variety of methods for an aPTT may be possible. In one preferred embodiment of the present invention the aPTT is measured in FIX depleted plasma samples. The improved FIX clotting activity of the a FIX mutant may be calculated in FIX-depleted plasma as specific FIX activity. Accordingly pdFIX and FIX-WT usually have specific FIX activity of about 100%. Any amino acid mutation leading to an increased specific FIX activity as compared to pdFIX or FIX-WT can be defined as increase. In a preferred embodiment of the present invention the increased activity of a rFIX mutant is at least 150 %, and more preferably more than 200 %. When using activated FIX or a FIX mutant the increased activity may be expressed alternatively as X-fold equivalent activity of pdFIXa. In a preferred embodiment of the present invention the increased activity of a rFIX mutant is at least a 10 fold equivalent activity of pdFIXa.

Another aspect of the present invention relates to a pharmaceutical composition comprising the rFIX mutant of the present invention having improved FIX clotting activity for treating a bleeding disorder associated with functional defects of FIX or deficiencies of FIX.

The pharmaceutical composition may further comprise an auxiliary agent, e.g. selected from the group consisting of a pharmaceutically acceptable carrier, diluent, salt, buffer, or excepient. Said pharmaceutical composition can be used for treating the above-defined bleeding disorders. The pharmaceutical composition of the invention may be a solution or a lyophilized product.

As used herein, the term "pharmaceutically acceptable" means approved by a regulatory agency of US or EU government or listed in the U.S. Pharmacopeia or other generally recognized pharmacopeia for use in animals, and more particularly in humans.

The pharmaceutical composition of the present invention can be used in a method for treating a bleeding disorder associated with functional defects of FIX or deficiencies of FIX comprising the step of administering the pharmaceutical composition comprising a rFIX mutant protein having an improved FIX clotting activity to a patient in need thereof.

The expression "bleeding disorder associated with functional defects of FIX or deficiencies of FIX" as used herein includes bleeding disorders, wherein the cause of the bleeding disorder may be selected from the group consisting of a shortened in *vivo*-half-life of FIX, altered binding properties of FIX, genetic defects of FIX, and a reduced plasma concentration of FIX. Genetic defects of FIX comprise for example deletions, additions and/or substitution of bases in the nucleotide sequence encoding FIX whose absence, presence and/or substitution, respectively, has a negative impact on the activity of FIX. FIX inhibitor development may be also responsible for defects in FIX function. In one example of the present invention, the bleeding disorder is hemophilia B.

The route of administration does not exhibit particular limitations, and in one embodiment the protein of the present invention may be administered by injection, such as intravenous, intramuscular, or intraperitoneal injection. In a preferred embodiment of the present invention the pharmaceutical composition may be administered intravenously.

The present invention will be further illustrated in the following examples, without any limitation thereto.
Figure 1 shows the structure of the FIX mutant cloning and expression vector
Figure 2 shows the determination of the amidolytic activity of mutated rFIXa proteins by cleavage of substrate pNAPEP0968
Figure 3 shows the aPTT assay of mutated rFIX proteins in FIX depleted plasma
Figure 4 shows the aPTT assay of activated mutant rFIX proteins in FIX-depleted plasma.

### EXAMPLES

### Example 1: Mutagenesis of FIX and Construction of FIX Expression Vectors

Publications referenced above discussing amino acid residues important for the activation of FX by FIX and own considerations were used for the construction of mutated FIX proteins. Two of the FIXa mutations are located on the 99-loop, known to contribute to substrate binding by forming the S2 and S4 substrate recognition site. The third FIXa mutation, Y177T, is placed adjacent to the S4 site. Finally three FIX-mutants with different mutation combinations FIX-Y94F/K98T (SEQ ID NO 4), FIX-Y94F/K98T/Y177F (SEQ ID NO 6), and FIX-Y94F/K98T/Y177F/I213V/E219G (SEQ ID NO 8) were cloned in addition to FIX-WT (SEQ ID NO 2). The respective SEQ ID NOs for the encoding nucleic acids are SEQ ID NO 3 (FIX-Y94F/K98T), SEQ ID NO 5 (FIX-Y94F/K98T/Y177F), SEQ ID NO 7 (FIX-Y94F/K98T/Y177F/I213V/E219G), and SEQ ID NO 1 (FIX-WT).

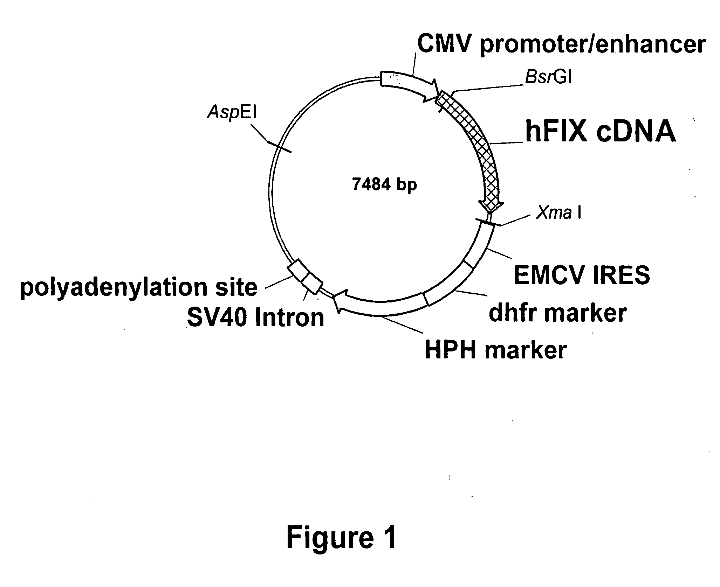

For the construction of the rFIX plasmids the FVIII cDNA from pCMVrFVIIIdB928/EDHPro (Herlitschka et al., J Biotechnol. 1998;61:165-73) was replaced by human FIX cDNA. The FIX cDNA encodes a polymorphism of human FIX leading to an amino acid exchange of Thr to Ala at position 194 in the activation peptide. The vector map of the plasmid is shown in Figure 1. A schematic of the transcription unit, containing the human cytomegalovirus (CMV) promoter/enhancer, the gene of interest (human FIX cDNA), an internal ribosomal entry site (EMCV IRES), the selection marker, the SV40 intron and the polyadenylation site is shown. The marker is a chimeric construct, consisting of the wild-type dihydrofolate reductase cDNA and the hygromycin phosphotransferase gene fused in frame.

For the construction of cDNA encoding FIX-Y94F/K98T, FIX-Y94F/K98T/Y177F, and FIX-Y94F/K98T/Y177F/I213V/E219G site-directed mutagenesis was performed using the QuickChange Site-Directed Mutagenesis Kit (Stratagene, La Jolla, CA, USA). All PCR reactions contained 125 ng sense primer, 125 ng antisense primer (Invitrogen, Carlsbad, CA, USA) and 5-50 ng dsDNA template, 2.5 units of PfuTurbo DNA polymerase and dNTPs in a final volume of 50 µL reaction buffer provided by the kit. After a pre-denaturation step of 1 minute at 95°C PfuTurbo DNA Polymerase was added followed by 18 cycles of 95°C for 30 seconds , 55°C for 60 seconds and 68°C for 12 minutes. The amplified product was incubated for 1 hour at 37°C with *Dpnl* to digest the methylated parental double stranded DNA before transformation into XL1-Blue Supercompetent Cells. For the construction of multiply mutated FIX cDNA this procedure was repeated with the according primers (Invitrogen) as shown in Table 1. The mutant FIX constructs were digested with restriction enzymes *Bsr*GI and Xmal (New England Biolabs, Ipswich, MA, USA) and subsequently ligated into the parental expression vector. Final FIX constructs were sequenced (Applied Biosystems Model 373A Sequencer Applied Biosystems, Foster City, CA) to confirm the mutations and were then linearized by *Asp*EI for transfection.

**Table 1: Primers for the mutagenesis of FIX constructs.**

| Mutations | Sense Primer | Antisense Primer |
|---|---|---|
| Y94F-K98T | 5´-cct cac cac aac ttc aat gca gct att aat acc tac aac cat gac-3´ (SEQ ID NO 9) | 5´-gtc atg gtt gta ggt att aat agc tgc att gaa gtt gtg gtg agg-3´ (SEQ ID NO 10) |
| Y177F | 5´-cac cat ctt taa caa cat gtt ctg-3´ (SEQ ID NO 11) | 5´-cag aac atg ttg tta aag atg gtg-3´ (SEQ ID NO 12) |
| I213V-E219G | 5´-ctg gaa ttg tga gct ggg gtg aag gct gtg caa tga aag gc-3´ (SEQ ID NO 13) | 5´-gcc ttt cat tgc aca gcc ttc acc cca gct cac aat tcc ag-3´ (SEQ ID NO 14) |

### Example 2: Expression of Recombinant FIX Proteins

All recombinant FIX proteins were expressed in 293 human embryo kidney cells (HEK293) using plasmids containing the human FIX-WT cDNA or mutated FIX cDNA and a hygromycin selection marker.

HEK 293 cells were grown in a mixture of Dulbecco's modified Eagle's Medium and F-12 medium supplemented with 5% fetal calf serum. Transfection was performed by lipofection using Lipofectamine™2000 reagent (Invitrogen). One to 2 days before transfection HEK 293 cells were seeded on 5 cm dishes to reach a confluence of 70-80 %. On the day of transfection the medium was exchanged 2 hours prior to the procedure. Six µg of FIX cDNA were transfected according to the recommended protocols. After 6 hours, fresh medium was added and the cells were cultured for 1 to 2 days before passaging into 15 cm dishes and selection of transfected cells with medium containing hygromycin at a concentration of 200 µg/mL. Two to 3 weeks later, the surviving foci were isolated into 24-well dishes in selective medium to produce stable cell lines. Each clone was grown to confluence in the presence of 5 µg/mL vitamin K1, and the secretion of FIX antigen into the medium was measured by an ELISA. FIX secreted by high-producer clones was additionally assayed in one-stage activated partial thromboplastin time assays (aPPT) and visualized on Western blots.

The best cell lines were selected for large-scale production in one-liter spinner flasks. Therefore cells were grown on 15 cm dishes to 90 % confluence, trypsinized and counted in a CASY cell counter with a 150 µm capillary (Schärfe Systems, Reutlingen, Germany). 500 mL stirred spinner flasks (60 rpm) were inoculated with 10⁶ cells/ mL in 200 mL medium without fetal calf serum and supplemented with 5 µg/mL vitamin K1 and 100 µg/mL hygromycin. The medium was expanded to a final volume of 1000 mL over the next few weeks depended on the rate of growth of the cells. The culture medium was collected twice weekly. Before storage at -20°C the culture medium was centrifuged and sterile filtrated (GP EXPRESS PLUS Membrane, SCGPT05RE, Millipore Corporation, Billerica, MS, USA) to remove cells and debris. The supernatant contained between 0.4 and 1 µg/mL rFIX antigen. rFIX-WT produced 2.6 µg/mL.

FIX antigen levels were determined by a double antibody sandwich ELISA. Therefore a sheep anti-human FIX affinity purified IgG (SAFIX-AP, Affinity Biologicals Inc., Ancaster, ON, Canada) was diluted in Tris-buffered saline (TBS, 25 mM Tris/HCl pH 7.4, 150 mM NaCl) to a concentration of 2 µg/mL and dispensed in 100 µL aliquots into the wells of a 96-well Nunc Maxisorp plate (Nunc, Roskilde, Denmark) which was then kept at 4°C over night. The plate was washed 3 times with TBST (TBS + 0.1% (v/v) Tween 20) followed by 1 hour blocking with 250 µL 3% non-fat dry milk powder (DMP) in TBS per well. The plate was then washed and 100 µL of FIX-dilution in 1 % DMP in TBST were distributed in the wells. Serial dilutions of pdFIX (Enzyme Research Laboratories, South Bend, IN, USA) were used as standard protein. The plate was incubated for 2 hours and then washed 5 times. Rabbit anti-human FIX IgG (Accurate Chemical & Scientific Corp., Westbury, NY, USA) was diluted in TBST/1 % DMP in a ratio of 1 to 6,000 and added to each well in 100 µL aliquots for 1 hour. After 5 washing steps 100 µL of a goat anti-rabbit IgG (H+L) horseradish peroxidase (HRP)-conjugate (Bio-Rad Laboratories, Hercules, CA, USA) diluted 1 to 3,000 in TBST/1 % DMP was added and incubated for 1 hour. Unbound conjugated antibody was removed by washing the plate 5 times. The addition of 100 µL 0.4 mg/mL o-phenylenediamine (OPD, Sigma, St. Louis, MO, USA) and 0.4 mg/mL urea hydrogen peroxide in 50 mM phosphate-citrate pH 5.0 started the color development. After an incubation time of 7.5 min the reaction was stopped by the addition of 50 µL 0.5 N H2SO₄. The absorbance at 492 nm was measured in an ELISA reader (Labsystems iEMF Reader MF, Vantaa, Finland).

### Example 3: Purification of Recombinant FIX Proteins

FIX proteins from serum-free conditioned medium were ultrafiltrated, purified by anion exchange chromatography, tandem-pseudoaffinity and affinity chromatography and polished by inactivation and removal of preactivated rFIX. All purification steps have been carried out on the chromatographic system Äkta™Explorer 100 Air (Amersham Biosciences, Umea, Sweden) at 4°C.

The collected frozen serum-free culture medium from rFIX expression was supplemented with 2 mM benzamidine and thawed at room temperature. The pooled supernatants of each rFIX construct were concentrated on a Sartorius UDF system using a 0.7 m² polyvinylidene-difluorid (PVDF) membrane with a 10 kDa molecular weight cut off. The system was run with a flow of 330 mL/min.

Recombinant FIX was captured from culture medium by anion exchange chromatography on Q-Sepharose Fast Flow in a XK26/60 column (Amersham). The matrix was equilibrated with 20 mM Tris/HCl pH 7.4 containing 0.1 % Tween 80, 2 mM benzamidine and 2 mM ethylenediamine tetraacetic acid (EDTA). UDF-filtrates supplemented with 2 mM EDTA were applied to the column at a rate of 23 cm/h. The column was reequilibrated and washed with 20 mM Tris/HCl pH 7.4, 0.1 % Tween 80, 200 mM NaCl, 2 mM benzamidine, 2 mM EDTA at 34 cm/h. The protein was eluted with 400 mM NaCl in equilibration buffer at the rate of 23 cm/h.

Tandem chromatography comprised a Ca²⁺-filtration of FIX on Q-Sepharose Fast Flow in a XK26/20 column followed by pseudoaffinity chromatography on Cellufine™ Sulfate (Chisso Corporation, Tokyo, Japan) in a XK26/20 column. The columns were switched on-line at sample application and reequilibration. Washing and elution was performed with the Cellufine™ Sulfate-column alone. The samples were equilibrated with 20 mM Tris/HCl pH 7.4, 100 mM NaCl, 0.1 % Tween 80, 2 mM benzamidine, and 20 mM CaCl₂. The elution-fraction of capture anion exchange chromatography diluted with equilibration-buffer containing 20 mM CaCl₂ with a conductivity of 16 mS/cm was applied onto the columns at 23 cm/h. After re-equilibration of both columns the Cellufine™ Sulfate column was washed with 20 mM Tris/HCl pH 7.4, 200 mM NaCl, 0.1 % Tween 80, 2 mM benzamidine and 1 mM CaCl₂. rFIX was eluted in a linear NaCl gradient from 200 to 1000 mM in washing buffer at a rate of 23 cm/h.

For affinity chromatography elution fractions of tandem chromatography were concentrated and buffer exchanged in Centriprep Ultracel YM-10 (Millipore, Bedford, MA, USA) at 2800 g and 8°C. The retentate, supplemented with 10 mM CaCl₂, 40 mM MgCl₂ and 1 mM benzamidine, was applied to a HR 16/10 column containing a Ca²⁺ dependent monoclonal antibody against human FIX light chain (American Diagnostica Inc., Stamford, CT, USA) coupled to NHS-acitivated Sepharose Fast Flow (Amersham) at 38 cm/h. The matrix was equilibrated before and after sample application with 25 mM Tris pH 7.4, 150 mM NaCl, 10 mM CaCl₂ and 10 mM MgCl₂. For the next washing step the salt concentration was increased to 1000 mM NaCl. rFIX was eluted with 25 mM Tris pH 7.4, 150 mM NaCl and 20 mM EDTA at a rate of 38 cm/h. The matrix was regenerated after each chromatography run with 25 mM Tris pH 7.4, 1000 mM NaCl and 20 mM EDTA.

The removal of preactivated rFIX was achieved by incubation of rFIX-solutions with a fifteen fold molar excess of the two biotinylated inhibitors Biotinyl-ε-aminocaproyl-D-Phe-Pro-Arg-chloromethylketone (BFPRCK, Bachem, Bubendorf, Switzerland) and Biotinyl-Glu-Gly-Arg-chloromethylketone (BEGRCK, Haematologic Technologies Inc., Essex Junction, VT, USA) over night at 4°C. FIX-Y94F-K98T was not treated with chloromethylketones.

rFIX fractions were supplemented with 0.1% ovalbumin and dialyzed in a Slide-A-Lyzer MWCO 10 kDa (Pierce, Rockford, IL, USA) against TBS before streptavidin-sepharose (Amersham) was added in excess to the chloromethylketones. Complexes of streptavidin-sepharose with biotinylated rFIX-chloromethylketones were formed at 4°C. These complexes were removed by 10 minute centrifugation at 4000 g and 4°C.

### Example 4: Determination of Amidolytic Activity of Mutated rFIXa Proteins by Cleavage of Substrate pNAPEP0968

The cleavage of H-D-Leu-Ph'Gly-Arg-pNA.2AcOH (pNAPEP0968, Biopep, Mauguio, France) by pdFIXa or a rFIXa mutant according to the invention was tested at 37°C in TBS containing 0.1% ovalbumin and 5 mM CaCl₂. Substrate hydrolysis was initiated by the addition of 50 µL of substrate solution to a 50 µL sample in a 96-well flat bottom microtiter plate (Greiner, Kremsmünster, Austria). Kinetic parameters of substrate hydrolysis were determined employing substrate concentrations ranging from 0 to 3.2 mM at an enzyme concentration of 75 nM. The release of p-nitroaniline was monitored at 405 nM in an ELISA reader (Labsystems iEMF Reader MF) for 30 minutes and the absorbance values were converted into molar concentrations using a molar extinction coefficient of 9.65 x 10³ M⁻¹cm⁻¹ for p-nitroaniline and a path length of 0.35 cm for a 100 µL volume.

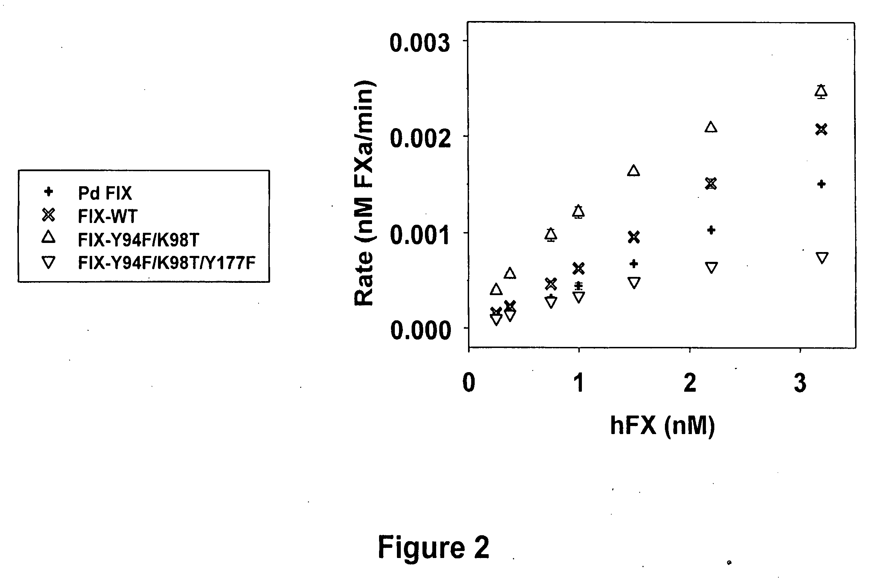

Data were fit to Michaelis-Menten kinetics. The amidolytic activity of pdFIXa, FIXa-WT and the mutated proteins FIXa-Y94F/K98T and FIXa-Y94F/K98T/Y177F is shown in Figure 2. From the data kinetic parameters were calculated (kcat/KM). As shown in Table 2 the hydrolysis of pNAPEP0968 by FIXa-WT, FIXa-Y94F/K98T/Y177F was similar to that of pdFIXa. In the case of FIXa-Y94F/K98T the catalytic specificity for this substrate was increased by a factor of 3.5 as compared to pdFIXa. Furthermore FIXa-Y94F/K98T/Y177F/I213V/E219G had an increased catalytic specificity by a factor of 2.4 (Table 2).

**Table 2: Kinetic parameters of pNAPEP0968 amidolysis.**

| | kcat/KM x 10³ (mM⁻¹min⁻¹) |
|---|---|
| pdFIXa | 6.3 |
| FIXa-WT | 8.8 |
| FIXa-Y94F/K98T | 22.2 |
| FIXa-Y94F/K98T/Y177F | 6.3 |
| FIXa-Y94F/K98T/Y177F/I213V/E219G | 15.2 |

### Example 5: Clotting Assays of FIX Proteins in Plasmatic Samples

Clotting assays, i.e. aPTT assays in plasmatic samples represent meaningful assays for testing the functional activity of the a mutant rFIX protein. Therefore pdFIX was serially diluted from 12.5 to 1000 mU/mL and the FII-FIX-FX reference standard from 3.125 to 400 mU/mL in imidazol buffer containing 1 % albumin (Baxter). Recombinant FIX-WT and FIX mutants were tested in 2 concentrations each of approximately 10 and 27 mU/mL. Fifty µL of these samples, 50 µL of plasma and 50 µL of STA-APTT reagent (Diagnostica Stago, Asniéres, France) were mixed and incubated at 37°C for 4 minutes. 50 µL of 25 mM CaCl₂ were added and time to clot formation was determined in an ACL10000 (Instrumentation Laboratory, Milano, Italy).

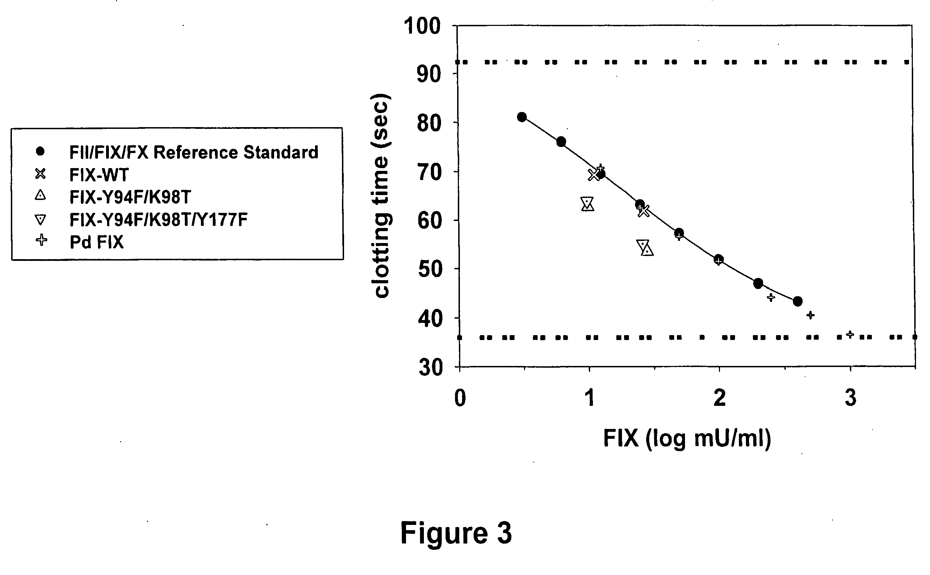

FIX proteins were tested in FIX-depleted plasma. Addition of FIX-WT and pdFIX to the plasma resulted in specific FIX activity of 97 and 108 %, respectively (Table 3). FIX-Y94F/K98T/Y177F/I213V/E219G had a comparable activity (Table 3). However, FIX-Y94F/K98T and FIX-Y94F/K98T/Y177F showed a concentration dependent decrease of clotting time (Figure 3). 0.14 µg/mL and 1.0 µg/mL FIX-Y94F/K98T reduced the clotting time to 62.7 and 53.5 seconds, respectively. FIX-Y94F/K98T/Y177F displayed a similar effect on clotting time with 63.9 and 55.1 seconds for 0.13 and 0.99 µg/mL mutant protein, respectively. The FII-FIX-FX reference standard titration, fitted to a 4-parameter algorithm, is indicated by a solid line in the figure. Clotting time of normal plasma (36 seconds) and that of FIX-depleted plasma (92 seconds) are indicated by dotted lines. Specific FIX activity is calculated according to the reference standard calibration. From the mutated proteins FIX-Y94F/K98T had the greatest effect with a specific activity of 249% whereas FIX-Y94F/K98T/Y177F resulted in a specific activity of 232%.

**Table 3: aPTT of FIX proteins in FIX-depleted plasma.**

| | Specific activity (%) |
|---|---|
| pdFIX | 97 |
| FIX-WT | 108 |
| FIX-Y94F/K98T | 249 |
| FIX-Y94F/K98T/Y177F | 232 |
| FIX-Y94F/K98T/Y177F/I213V/E219G | 90 |

### Example 6: Clotting Assays of Activated Mutant FIX Proteins in FIX-depleted Plasmatic Samples

In the clotting assay described above FIX is directly activated by FXIa before it can activate FX. A poor activity of a rFIX mutant in the clotting assay could therefore reflect impaired activation by FXIa or a low activity in FX activation. To further investigate the FX activation potential of the rFIX mutants without an influence of activation rates by FXIa the clotting activity of pre-activated rFIX mutants was determined in clotting assays in FIX-depleted plasma. For activation pdFIX and rFIX mutants were diluted to 25 µg/mL in TBS containing 5 mM CaCl₂ and 0.1 % ovalbumin. FIX activation was started by the addition of pdFXIa at a molar enzyme substrate ratio of 1 to 500 at 37°C. FXIa was removed with affinity purified goat anti-FXI IgG bound to protein G sepharose.

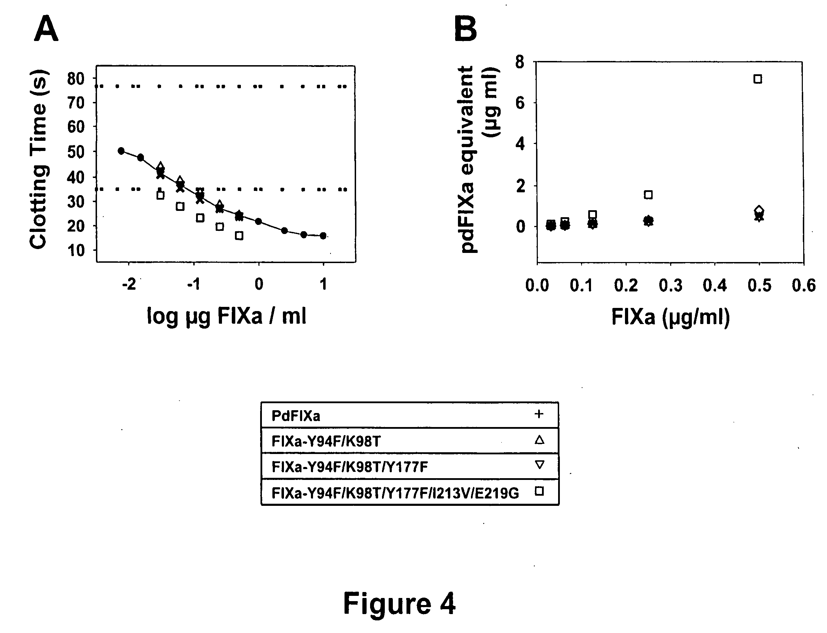

APTT was measured at concentrations of FIXa proteins between 0.03125 and 0.5 µg/mL. PdFIXa was the standard. 50 µL FIX-depleted plasma and 50 µL activated FIXa were mixed with 50 µL aPTT-reagent for 2 minutes at 37°C. Clotting time measurement was started by addition of 50 µL 25 mM CaCl₂. Titration with a pdFIXa standard, fitted to a four-parameter algorithm, is shown in black (Figure 4A). Black dotted lines show clotting times of FIX-depleted plasma and of normal plasma.
In Figure 4 B pdFIXa equivalent activity was calculated using the pdFIXa calibration curve (0.0625-10 µg/mL). All FIXa variants showed similar activity as pdFIXa except FIXa-Y94F/K98T/Y177F/I213V/E219G which exhibited a 14.4-fold higher activity than pdFIXa. Table 4 shows the pdFIXa equivalent activity given for 0.5 µg/mL of FIXa proteins.

**Table 4. APTT of activated pdFIX and rFIX proteins in FIX-depleted plasma.**

| | µg/mL pdFIXa activity |
|---|---|
| pdFIXa | 1.1 |
| FIXa-Y94F/K98T | 0.9 |
| FIXa-Y94F/K98T/Y177F | 1.0 |
| FIXa-Y94F/K98T/Y177F/I213V/E219G | 14.4 |

This invention shows that a rationally designed rFIX protein with combinations of mutations can improve FIX clotting activity. Therefore these molecules can be used for treatment of bleeding disorder associated with functional defects of FIX or deficiencies of FIX.

### Sequence Listings from File Reference #8010

SEQ ID NO 1
   FIXWT 
atgcagcgcg tgaacatgat catggcagaa tcaccaggcc tcatcaccat ctgcctctta 60
ggatatctac tcagtgctga atgtacagtt tttcttgatc atgaaaacgc caacaaaatt 120
ctgaatcggc caaagaggta taattcaggt aaattggaag agtttgttca agggaacctt 180
gagagagaat gtatggaaga aaagtgtagt tttgaagaag cacgagaagt ttttgaaaac 240
actgaaagaa caactgaatt ttggaagcag tatgttgatg gagatcagtg tgagtccaat 300
ccatgtttaa atggcggcag ttgcaaggat gacattaatt cctatgaatg ttggtgtccc 360
tttggatttg aaggaaagaa ctgtgaatta gatgtaacat gtaacattaa gaatggcaga 420
tgcgagcagt tttgtaaaaa tagtgctgat aacaaggtgg tttgctcctg tactgaggga 480
tatcgacttg cagaaaacca gaagtcctgt gaaccagcag tgccatttcc atgtggaaga 540
gtttctgttt cacaaacttc taagctcacc cgtgctgagg ctgtttttcc tgatgtggac 600
tatgtaaatt ctactgaagc tgaaaccatt ttggataaca tcactcaaag cacccaatca 660
tttaatgact tcactcgggt tgttggtgga gaagatgcca aaccaggtca attcccttgg 720
caggttgttt tgaatggtaa agttgatgca ttctgtggag gctctatcgt taatgaaaaa 780
tggattgtaa ctgctgccca ctgtgttgaa actggtgtta aaattacagt tgtcgcaggt 840
gaacataata ttgaggagac agaacataca gagcaaaagc gaaatgtgat tcgaattatt 900
cctcaccaca actacaatgc agctattaat aagtacaacc atgacattgc ccttctggaa 960
ctggacgaac ccttagtgct aaacagctac gttacaccta tttgcattgc tgacaaggaa 1020
tacacgaaca tcttcctcaa atttggatct ggctatgtaa gtggctgggg aagagtcttc 1080
cacaaaggga gatcagcttt agttcttcag taccttagag ttccacttgt tgaccgagcc 1140
acatgtcttc gatctacaaa gttcaccatc tataacaaca tgttctgtgc tggcttccat 1200
gaaggaggta gagattcatg tcaaggagat agtgggggac cccatgttac tgaagtggaa 1260
gggaccagtt tcttaactgg aattattagc tggggtgaag agtgtgcaat gaaaggcaaa 1320
tatggaatat ataccaaggt atcccggtat gtcaactgga ttaaggaaaa aacaaagctc 1380
acttaa 1386
SEQ ID NO 2
   FIXWT 
 60
 120
 180
 240
 300
 360
 420
 461
SEQ ID NO 3
   FIX94-98 
atgcagcgcg tgaacatgat catggcagaa tcaccaggcc tcatcaccat ctgcctctta 60
ggatatctac tcagtgctga atgtacagtt tttcttgatc atgaaaacgc caacaaaatt 120
ctgaatcggc caaagaggta taattcaggt aaattggaag agtttgttca agggaacctt 180
gagagagaat gtatggaaga aaagtgtagt tttgaagaag cacgagaagt ttttgaaaac 240
actgaaagaa caactgaatt ttggaagcag tatgttgatg gagatcagtg tgagtccaat 300
ccatgtttaa atggcggcag ttgcaaggat gacattaatt cctatgaatg ttggtgtccc 360
tttggatttg aaggaaagaa ctgtgaatta gatgtaacat gtaacattaa gaatggcaga 420
tgcgagcagt tttgtaaaaa tagtgctgat aacaaggtgg tttgctcctg tactgaggga 480
tatcgacttg cagaaaacca gaagtcctgt gaaccagcag tgccatttcc atgtggaaga 540
gtttctgttt cacaaacttc taagctcacc cgtgctgagg ctgtttttcc tgatgtggac 600
tatgtaaatt ctactgaagc tgaaaccatt ttggataaca tcactcaaag cacccaatca 660
tttaatgact tcactcgggt tgttggtgga gaagatgcca aaccaggtca attcccttgg 720
caggttgttt tgaatggtaa agttgatgca ttctgtggag gctctatcgt taatgaaaaa 780
tggattgtaa ctgctgccca ctgtgttgaa actggtgtta aaattacagt tgtcgcaggt 840
gaacataata ttgaggagac agaacataca gagcaaaagc gaaatgtgat tcgaattatt 900
cctcaccaca acttcaatgc agctattaat acctacaacc atgacattgc ccttctggaa 960
ctggacgaac ccttagtgct aaacagctac gttacaccta tttgcattgc tgacaaggaa 1020
tacacgaaca tcttcctcaa atttggatct ggctatgtaa gtggctgggg aagagtcttc 1080
 
cacaaaggga gatcagcttt agttcttcag taccttagag ttccacttgt tgaccgagcc 1140
acatgtcttc gatctacaaa gttcaccatc tataacaaca tgttctgtgc tggcttccat 1200
gaaggaggta gagattcatg tcaaggagat agtgggggac cccatgttac tgaagtggaa 1260
gggaccagtt tcttaactgg aattattagc tggggtgaag agtgtgcaat gaaaggcaaa 1320
tatggaatat ataccaaggt atcccggtat gtcaactgga ttaaggaaaa aacaaagctc 1380
acttaatga 1389
SEQ ID NO 4
   FIX94-98 
MQRVNMIMAE SPGLITICLL GYLLSAECTV FLDHENANKI LNRPKRYNSG KLEEFVQGNL 60
ERECMEEKCS FEEAREVFEN TERTTEFWKQ YVDGDQCESN PCLNGGSCKD DINSYECWCP 120
FGFEGKNCEL DVTCNIKNGR CEQFCKNSAD NKVVCSCTEG YRLAENQKSC EPAVPFPCGR 180
VSVSQTSKLT RAEAVFPDVD YVNSTEAETI LDNITQSTQS FNDFTRVVGG EDAKPGQFPW 240
QVVLNGKVDA FCGGSIVNEK WIVTAAHCVE TGVKITVVAG EHNIEETEHT EQKRNVIRII 300
PHHNFNAAIN TYNHDIALLE LDEPLVLNSY VTPICIADKE YTNIFLKFGS GYVSGWGRVF 360
HKGRSALVLQ YLRVPLVDRA TCLRSTKFTI YNNMFCAGFH EGGRDSCQGD SGGPHVTEVE 420
GTSFLTGIIS WGEECAMKGK YGIYTKVSRY VNWIKEKTKL T 461
SEQ ID NO 5
   FIX94-98-177 
atgcagcgcg tgaacatgat catggcagaa tcaccaggcc tcatcaccat ctgcctctta 60
ggatatctac tcagtgctga atgtacagtt tttcttgatc atgaaaacgc caacaaaatt 120
ctgaatcggc caaagaggta taattcaggt aaattggaag agtttgttca agggaacctt 180
gagagagaat gtatggaaga aaagtgtagt tttgaagaag cacgagaagt ttttgaaaac 240
actgaaagaa caactgaatt ttggaagcag tatgttgatg gagatcagtg tgagtccaat 300
ccatgtttaa atggcggcag ttgcaaggat gacattaatt cctatgaatg ttggtgtccc 360
tttggatttg aaggaaagaa ctgtgaatta gatgtaacat gtaacattaa gaatggcaga 420
tgcgagcagt tttgtaaaaa tagtgctgat aacaaggtgg tttgctcctg tactgaggga 480
tatcgacttg cagaaaacca gaagtcctgt gaaccagcag tgccatttcc atgtggaaga 540
gtttctgttt cacaaacttc taagctcacc cgtgctgagg ctgtttttcc tgatgtggac 600
tatgtaaatt ctactgaagc tgaaaccatt ttggataaca tcactcaaag cacccaatca 660
tttaatgact tcactcgggt tgttggtgga gaagatgcca aaccaggtca attcccttgg 720
caggttgttt tgaatggtaa agttgatgca ttctgtggag gctctatcgt taatgaaaaa 780
tggattgtaa ctgctgccca ctgtgttgaa actggtgtta aaattacagt tgtcgcaggt 840
gaacataata ttgaggagac agaacataca gagcaaaagc gaaatgtgat tcgaattatt 900
cctcaccaca acttcaatgc agctattaat acctacaacc atgacattgc ccttctggaa 960
ctggacgaac ccttagtgct aaacagctac gttacaccta tttgcattgc tgacaaggaa 1020
tacacgaaca tcttcctcaa atttggatct ggctatgtaa gtggctgggg aagagtcttc 1080
cacaaaggga gatcagcttt agttcttcag taccttagag ttccacttgt tgaccgagcc 1140
acatgtcttc gatctacaaa gttcaccatc tttaacaaca tgttctgtgc tggcttccat 1200
gaaggaggta gagattcatg tcaaggagat agtgggggac cccatgttac tgaagtggaa 1260
gggaccagtt tcttaactgg aattattagc tggggtgaag agtgtgcaat gaaaggcaaa 1320
tatggaatat ataccaaggt atcccggtat gtcaactgga ttaaggaaaa aacaaagctc 1380
acttaatga 1389
SEQ ID NO 6
   FIX94-98-177 
MQRVNMIMAE SPGLITICLL GYLLSAECTV FLDHENANKI LNRPKRYNSG KLEEFVQGNL 60
ERECMEEKCS FEEAREVFEN TERTTEFWKQ YVDGDQCESN PCLNGGSCKD DINSYECWCP 120
FGFEGKNCEL DVTCNIKNGR CEQFCKNSAD NKVVCSCTEG YRLAENQKSC EPAVPFPCGR 180
VSVSQTSKLT RAEAVFPDVD YVNSTEAETI LDNITQSTQS FNDFTRVVGG EDAKPGQFPW 240
QVVLNGKVDA FCGGSIVNEK WIVTAAHCVE TGVKITVVAG EHNIEETEHT EQKRNVIRII 300
PHHNFNAAIN TYNHDIALLE LDEPLVLNSY VTPICIADKE YTNIFLKFGS GYVSGWGRVF 360
HKGRSALVLQ YLRVPLVDRA TCLRSTKFTI FNNMFCAGFH EGGRDSCQGD SGGPHVTEVE 420
GTSFLTGIIS WGEECAMKGK YGIYTKVSRY VNWIKEKTKL T 461
SEQ ID NO 7
   FIX94-98-177-213-219 atgcagcgcg tgaacatgat catggcagaa tcaccaggcc tcatcaccat ctgcctctta 60 
ggatatctac tcagtgctga atgtacagtt tttcttgatc atgaaaacgc caacaaaatt 120
ctgaatcggc caaagaggta taattcaggt aaattggaag agtttgttca agggaacctt 180
gagagagaat gtatggaaga aaagtgtagt tttgaagaag cacgagaagt ttttgaaaac 240
actgaaagaa caactgaatt ttggaagcag tatgttgatg gagatcagtg tgagtccaat 300
ccatgtttaa atggcggcag ttgcaaggat gacattaatt cctatgaatg ttggtgtccc 360
tttggatttg aaggaaagaa ctgtgaatta gatgtaacat gtaacattaa gaatggcaga 420
tgcgagcagt tttgtaaaaa tagtgctgat aacaaggtgg tttgctcctg tactgaggga 480
tatcgacttg cagaaaacca gaagtcctgt gaaccagcag tgccatttcc atgtggaaga 540
gtttctgttt cacaaacttc taagctcacc cgtgctgagg ctgtttttcc tgatgtggac 600
tatgtaaatt ctactgaagc tgaaaccatt ttggataaca tcactcaaag cacccaatca 660
tttaatgact tcactcgggt tgttggtgga gaagatgcca aaccaggtca attcccttgg 720
caggttgttt tgaatggtaa agttgatgca ttctgtggag gctctatcgt taatgaaaaa 780
tggattgtaa ctgctgccca ctgtgttgaa actggtgtta aaattacagt tgtcgcaggt 840
gaacataata ttgaggagac agaacataca gagcaaaagc gaaatgtgat tcgaattatt 900
cctcaccaca acttcaatgc agctattaat acctacaacc atgacattgc ccttctggaa 960
ctggacgaac ccttagtgct aaacagctac gttacaccta tttgcattgc tgacaaggaa 1020
tacacgaaca tcttcctcaa atttggatct ggctatgtaa gtggctgggg aagagtcttc 1080
cacaaaggga gatcagcttt agttcttcag taccttagag ttccacttgt tgaccgagcc 1140
acatgtcttc gatctacaaa gttcaccatc tttaacaaca tgttctgtgc tggcttccat 1200
gaaggaggta gagattcatg tcaaggagat agtgggggac cccatgttac tgaagtggaa 1260
gggaccagtt tcttaactgg aattgtgagc tggggtgaag gctgtgcaat gaaaggcaaa 1320
tatggaatat ataccaaggt atcccggtat gtcaactgga ttaaggaaaa aacaaagctc 1380
acttaatga 1389
SEQ ID NO 8
   FIX94-98-177-213-219

MQRVNMIMAE SPGLITICLL GYLLSAECTV FLDHENANKI LNRPKRYNSG KLEEFVQGNL 60
ERECMEEKCS FEEAREVFEN TERTTEFWKQ YVDGDQCESN PCLNGGSCKD DINSYECWCP 120
FGFEGKNCEL DVTCNIKNGR CEQFCKNSAD NKVVCSCTEG YRLAENQKSC EPAVPFPCGR 180
VSVSQTSKLT RAEAVFPDVD YVNSTEAETI LDNITQSTQS FNDFTRVVGG EDAKPGQFPW 240
QVVLNGKVDA FCGGSIVNEK WIVTAAHCVE TGVKITVVAG EHNIEETEHT EQKRNVIRII 300
PHHNFNAAIN TYNHDIALLE LDEPLVLNSY VTPICIADKE YTNIFLKFGS GYVSGWGRVF 360
HKGRSALVLQ YLRVPLVDRA TCLRSTKFTI FNNMFCAGFH EGGRDSCQGD SGGPHVTEVE 420
GTSFLTGIVS WGEGCAMKGK YGIYTKVSRY VNWIKEKTKL T 461

SEQ ID NO 9
   Y94F-K98Ts
   cctcaccaca acttcaatgc agctattaat acctacaacc atgac 45
SEQ ID NO 10
   Y94F-K98Tas
   gtcatggttg taggtattaa tagctgcatt gaagttgtgg tgagg 45
SEQ ID NO 11
   Y177FS
   caccatcttt aacaacatgt tctg 24
SEQ ID NO 12
   Y177Fas
   cagaacatgt tgttaaagat ggtg 24
SEQ ID NO 13
   I213V-E219Gs
   ctggaattgt gagctggggt gaaggctgtg caatgaaagg c 41
SEQ ID NO 14
   I213V-E219Gas
   gcctttcatt gcacagcctt caccccagct cacaattcca g 41

## Claims

1. A recombinant blood coagulation factor IX (rFIX) mutant having improved FIX clotting activity, wherein the amino acid sequence of mutated rFIX is SEQ ID NO 4 (FIX-Y94F/K98T).

2. A mutant according to claim 1, wherein the amino acid sequence of mutated rFIX is encoded by the nucleic acid sequence SEQ ID NO 3 (FIX-Y94F/K98T).

3. A rFIX mutant nucleic acid sequence with SEQ ID NO 3 (FIX-Y94F/K98T).

4. A vector comprising a nucleic acid sequence encoding a mutant according to claim 3.

5. A mammalian cell line comprising a vector of claim 4 expressing a mutant according to claim 1.

6. A cell line according to claim 5, wherein the cell line is HEK 293.

7. A method for production of a rFIX mutant according to claim 1 comprising a) generation of said rFIX, b) cloning of said rFIX, c) expression of said rFIX in a cell line, and d) purification of said rFIX.

8. A pharmaceutical composition comprising a FIX mutant according to claim 1.

9. A pharmaceutical composition according to claim 8 for use in treating a bleeding disorder associated with functional defects of FIX or deficiencies of FIX.

10. The pharmaceutical composition for use according to claim 9, wherein the bleeding disorder is hemophilia B.

## Patentansprüche

1. Rekombinante Mutante des Blutgerinnungsfaktors IX (rFIX), die verbesserte FIX Gerinnungsaktivität aufweist, wobei die Aminosäuresequenz des mutierten rFIX SEQ ID No. 4 ist.

2. Mutante nach Anspruch 1, wobei die Aminosäuresequenz des mutierten rFIX durch die Nukleinsäuresequenz SEQ ID No. 3 (FIX-Y94F/K98T) kodiert wird.

3. Nukleinsäuresequenz einer rFIX Mutante mit SEQ ID No. 3 (FIX-Y94F/K98T).

4. Vektor, umfassend eine Nukleinsäuresequenz, die eine Mutante nach Anspruch 3 kodiert.

5. Säugerzelllinie, umfassend einen Vektor nach Anspruch 4, die eine Mutante nach Anspruch 1 exprimiert.

6. Zelllinie nach Anspruch 5, wobei die Zelllinie HEK 293 ist.

7. Verfahren zur Herstellung einer rFIX-Mutante nach Anspruch 1, umfassend a) Erzeugen des rFIX, b) Klonieren des rFIX, c) Exprimieren des rFIX in einer Zelllinie und d) Aufreinigen des rFIX.

8. Pharmazeutische Zusammensetzung, umfassend eine FIX-Mutante nach Anspruch 1.

9. Pharmazeutische Zusammensetzung nach Anspruch 8 zur Verwendung in der Behandlung einer Blutungsstörung, die mit funktionellen Defekten von FIX oder Defizienzen von FIX in Verbindung steht.

10. Pharmazeutische Zusammensetzung zur Verwendung nach Anspruch 9, wobei die Blutungsstörung Hämophilie B ist.

## Revendications

1. Mutant d'un facteur IX de coagulation recombinant (rFIX) présentant une amélioration de l'activité de coagulation du FIX, dans lequel la séquence d'acides aminés du rFIX muté est la SEQ ID N° 4 (FIX-Y94F/K98T).

2. Mutant selon la revendication 1, dans lequel la séquence d'acides aminés du rFIX muté est codée par la séquence d'acide nucléique SEQ ID N° 3 (FIX-Y94F/K98T).

3. Séquence d'acide nucléique d'un mutant du rFIX de SEQ ID N° 3 (FIX-Y94F/K98T).

4. Vecteur comprenant une séquence d'acide nucléique codant pour un mutant selon la revendication 3.

5. Lignée cellulaire de mammifère comprenant un vecteur selon la revendication 4 exprimant un mutant selon la revendication 1.

6. Lignée cellulaire selon la revendication 5, dans laquelle la lignée cellulaire est HEK 293.

7. Procédé de production d'un mutant du rFIX selon la revendication 1, comprenant a) la génération dudit rFIX, b) le clonage dudit rFIX, c) l'expression dudit rFIX dans une lignée cellulaire, et d) la purification dudit rFIX.

8. Composition pharmaceutique comprenant un mutant du FIX selon la revendication 1.

9. Composition pharmaceutique selon la revendication 8, destinée à être utilisée dans le traitement d'un trouble de la coagulation associé à des anomalies fonctionnelles du FIX ou à des déficits en FIX.

10. Composition pharmaceutique destinée à être utilisée selon la revendication 9, dans laquelle le trouble de la coagulation est l'hémophilie B.
